# EUROPEAN PATENT APPLICATION

(11) **EP 4 074 305 A1**
(43) Date of publication of application: **19.10.2022**
(21) Application number: 20898902.0
(22) Date of filing: 28.09.2020
(51) Int. Cl.: A61K 9/02, A61K 9/06, A61K 9/107, A61K 31/167, A61K 31/4422, A61K 31/495, A61K 47/00, A61P 1/00

(54) **PHARMACOLOGICAL COMPOSITION FOR TREATING PROCTOLOGICAL DISEASES (VARIANTS)**

(30) Priority: 13.12.2019 RU 2019141455
(71) Applicant: Barannikov, Alexander Evgenevich, Moscow, 121165 (RU)
(72) Inventor: Barannikov, Alexander Evgenevich, Moscow, 121165 (RU)
(74) Representative: Chimini, Francesco
(86) International application number: PCT/RU2020/000498
(87) International publication number: WO 2021/118400

(57) **Abstract**

This invention relates to medicine and more particularly to pharmacological compositions for treating proctologic diseases. The pharmacological composition comprises 3.3-7.5 wt% methyluracil, 0.13-0.3 wt% nifedipine, 1.3-5 wt% lidocaine, a pharmacologically acceptable carrier - rest. The pharmacological composition could be in the form of gel, or ointment, or cream, or suppositories, or suspended gel, or lotion, or emulsion.

## Description

### Field of the Invention

This invention relates to medicine and more particularly to pharmacological compositions for treating proctologic diseases.

The proposed pharmacological composition can be used for treating chronic hemorrhoids, acute hemorrhoidal manifestations (all stages), anal fissures, traumatic injuries of anus and rectum, sphincter, post-operative care of persons completed a treatment of hemorrhoids, polyps, papillomas, condylomata by surgical methods, pathological changes of hemorrhoidal boluses, as anesthetic and relaxing agent for anorectal area, anal mucous membrane irritation, sphincter spasmolysis, in mono- and complex therapy of proctologic diseases, such as: proctitis, periproctitis, rectum cancer, anal cancer, proctosigmoiditis, anal fistulas, anal inflammatory processes, cryptitis, megacolon, rectal fibroma.

All clinical aspects of pathological changes of hemorrhoidal boluses (rectal diseases) are referred to hemorrhoids. About 118 persons suffering from hemorrhoids are accounted for 1000 adults. In Russia about 5000 operations per year are carried out in patients with chronic hemorrhoids diagnosis. it is not uncommon due to prevalence of chronic constipation, and the majority of patients are diagnosed with these two diseases at the same time. There two stages of rectal diseases: acute and chronic. Manifestations of the disease are minimal at the chronic stage, patients retain ability to work, application of simple measures enables to forget the disease completely. Acute stage often requires hospitalization, at proper treatment the inflammation ceases, and the disease again changes into a chronic stage. Rectal diseases often begin with antecedent period. These antecedents are: anus discomfort, light pruritus, some difficulty during defecation, and this period lasts from some months to several years. Then, defecation bleeding of varying intensity occur - from blood stains on fecal masses to major bleeding. Along with bleeding prolapsed internal hemorrhoids is a common complaint.

Anal fissure - spontaneously occurring linear or ellipsoid defect of anus mucous membrane and anoderm (junction of mucous membrane and skin) ranks third (up to 11-13%) in patient appealability in the general structure of coloproctological profile. Anal fissure disease accounts for 20-30 cases for 1000 adults, proportion of such patients in the indoor patient structure is 3-5%. There are two forms of the disease, which are the stages of one process and have clinical progression features: acute anal fissure and chronic anal fissure. Acute anal fissure has a slit-like form with smooth even borders, its bottom is a muscular tissue of anus internal sphincter. Generally, the fissure is in midline, more often on the rear wall of anal canal, more rarely - on the front wall. There is a major danger of mucous membrane injury at defecation, and there are the worst conditions of blood supply in these places. Acute fissure is characterized by a triad of three complaints: pain during defecation, sphincter spasm, anus bloody discharge. Pain, generally, is very intensive and may persist for a long time. Severe pains force a patient to suspend defecation, that commonly results in constipation. The second permanent disease symptom - anal sphincter spasm is also associated with pain. When occurred during defecation it could last for days up until the next defecation. This particular symptom has a dominant role in disease process, completing the vicious loop - pain causes sphincter spasm, spasm intensifies pain and inhibits healing of fissure. Continuous injuring the mucous membrane defect by fecal masses is accompanied by rectal bleeding. Over time acute anal fissure transforms to a chronic one. Its bottom and borders are covered by granulation tissue with fibrinous pellicle. At long-lasting disease course there is scar tissue proliferation along the fissure borders with induration thereof. In the area of outer fissure border an excessive tissue portion - anal sentinel pile is developed. Such progression of scar tissue in the inner fissure border could cause anal skin tag. Further, tissues in the fissure area are subjected to nutritional disorder.

### Prior Art

There is a great number of patent documents (RU2457806, RU2436537, RU2427331, RU2011119393, RU2332176, RU2296528, RU92006917), which disclose pharmaceutical composition for treating various manifestations of hemorrhoids, and also for reliving rectum painful sensations, while the majority of the patents refer to surgical treatment of the above diseases. However, the selection priority in noninvasive treatment methods is inarguable.

Natural extract-based compositions (RU2470651, RU2455978, RU2011133881, RU2225213, RU2207137, RU98101150) are quite common, however, action of such medications is slow, gradual and not able to relive painful sensations quickly, without using analgesics and spasmolytics, moreover, cases of strong allergic reactions are not uncommon. Plant extracts, generally, have variable composition and could differ depending on plant harvest period, changes of plant material extraction and processing techniques. Commonly, companies register them not as drugs but as biologically active additives.

There are known antivaricose combination homeopathic products (RU2256462, RU2253471, RU2143271), which are used for treating complicated and noncomplicated aggregate of varicose symptoms, however, this concept in medicine is still open to argument on efficiency aspect, and homeopathic products are not pharmaceutical.

For the past two decades special attention has been given to sphincter relaxants: calcium antagonist-based medications (calcium channel blockers) and nitric oxide (NO) donors in treating rectum varicose diseases. The best known calcium channel blockers are nifedipine and diltiazem. Effect of nifedipine was first studied and described by Chrysos in 1996 (Chrysos E. et al, Effect of nifedipine on recoanal motility, Dis. Colon. Rectam, 1996, 39, 212-216), further, Carapeti studied a possibility of local clinical use of diltiazem (Carapeti E.A. et al, Toppical diltiazem and bethanecol decrease anal sphincter pressure and heal anal fissures without side effect. Dis. Colon Rectum, 2000, 43, 1359-1362). A great number of research publications have been dedicated to use of nitrates as NO donors for reducing pressure in affected region. The most commonly used medications are nitroglycerin, isosorbide di- and mononitrates, L-arginin (Loder A.F. et al, Reversible chemical sphinterotomy by local application glyceryl trinitrate, Br. J. Surg., 1994, 81, 1386-1389; Guillemot F. et al, Action in situ nitrogligerin on upper anal canal pressure of patients with terminal constipation. A pilot study., Dis. Colon Rectum, 1993, 36, 372-376; Schouten W.R. et al, Pathophysialogical aspects and clinical outcome of intra-anal application of isosorbide dinitrate in patients with chronic anal fissure, Gut, 1996, 39, 465-469; Griffin N. et al, Topical L-arginin gel lowers resting anal pressure: possible treatment for anal fissure, Dis. Colon Rectum, 2002, 45, 1332-1336).

There are patent documents disclosing pharmacological compositions comprising sphincter relaxants. Japanese patent B JP2002356425 discloses a composition consisting of 0.5-10 wt% nifedipine, which can also comprise bethanechol. US patent US8048875 also discloses a composition based on 2 wt% diltiazem, a calcium channel blocker, or its salts for treating diseases related to high pressure or spasms in anal region. US patent US7189761 discloses a composition based on nitric oxide donors with 0.01 wt% to 10 wt .% active substance for treating anorectal diseases. The international application WO2002/03973 discloses a formulation based on isosorbide dinitrate with possible adding a calcium channel blocker (nifedipine) as an additional active ingredient. US patent US20110263568 discloses a calcium antagonist-based medication with an option of presence of nitric oxide donors. Some papers disclose mixture compositions based on cholinergic agonists and calcium channel blockers (US20040028752, US20130079332).

The disadvantage of the above patents is that the said sphincter relaxants do not comprise anesthetics for fast local prevention of acute and chronic painful sensations associated with hemorrhoid inflammation, injury of anus mucous membrane and other diseases and pathological states of anorectal area, and also that their main components do not comprise a regenerant promoting healing processes.

The closest prior art for the proposed composition is the composition for treating proctologic diseases (RU 2592366) consisting of 3.3-7.5 wt% methyluracil, 0.13-0.3 wt% nifedipine, 1.3-3 wt% lidocaine, and also 0.13-0.3 wt% isosorbide dinitrate. However, the known medication is difficult-to-make and rather expensive. Besides, pain relieving effect of the known composition is insufficiently expressed.

Thus, there is a need for producing a composition for treating proctologic diseases, which has less complex composition, simple to produce, with minimal reduction of therapeutic effect and having more stronger analgesic effect.

### Disclosure of Technical Solution

The task is solved by the proposed pharmacological composition for treating proctologic diseases, which comprises 3.3-7.5 wt% methyluracil regenerant (dioxomethyltetrahydropyrimidine), 0,13-0,3 wt% calcium channel blocker - calcium antagonist, 1,3-5 wt% local anesthetic, a pharmacologically acceptable carrier - rest.

The pharmacological composition uses nifedipine as calcium channel blocker.

As a local anesthetic the pharmacological composition uses proxymetacaine, bupivacaine, benzocaine, bromoaniline dimethylamino propane acid, articaine, oxybuprocaine, lidocaine, tetracaine, dyclonine, dimethylaminoethyl ester of n-butylamino benzoic acid, mepivacaine, ropivacaine, procaine, trimecaine or a mixture thereof.

Preferably the pharmacological composition for treating proctologic diseases comprises 5 wt% methyluracil, 0.2 wt% nifedipine, 3.5 wt% lidocaine, a pharmacologically acceptable carrier - rest.

Moreover, both variants of the pharmacological composition could be in the form of gel, or ointment, or cream, or suppositories, or suspended gel, or lotion, or emulsion.

The technical result of the claimed invention is simplifying the manufacture of a composition for treating hemorrhoids and anal fissures, and intensifying the analgesic effect.

Pharmacological effect of methyluracil (dioxomethyltetrahydropyrimidine) - anti-inflammatory, leukopoietic, regeneration stimulating, reparative, wound healing, immunostimulating. The medication has anabolic and anticatabolic activity, accelerates cellular regeneration processes, accelerates wound healing, stimulates protective host and humoral factors. It also produces anti-inflammatory effect. A characteristic feature of this series compounds is stimulation of erythro- and specifically leukopoiesis, therefore, they normally belong to the leukopoiesis stimulant group. As a leukopoiesis stimulant, methyluracil is prescribed at neutropenic angina, alimentary toxic aleukia, chronic benzene intoxication, leukopenia resulted from cancer chemotherapy, at radiation and radiotherapy, and other states accompanied by leukopenia. It is necessary to take into account, that it is reasonable to apply methyluracil, as the other leukopoiesis stimulants, at mild forms of leukopenia. At moderate injuries application of hematopoietic stimulants is indicated only in case of reinitiation of disturbed blood cell regeneration. It is also prescribed at sluggish wounds, burns, bone fractures. There are data on methyluracil efficiency at gastroduodenal ulcer and chronic gastritis. It is assumed that therapeutic effect is associated with normalization of nucleic exchange in mucous membrane.

The following compounds can be used as a calcium channel blocker (calcium antagonist): amlodipine, anipamil, aranidipine, azelnidipine, barnidipine, benidipine, bepridil, darodipine, diltiazem, cilnidipine, clevidipine, isradipine, efonidipine, felodipine, lacidipine, lidofazin, lercanidipine, manidipine, mepirodipin, nicardipine, nifedipine, niludipin, nilvadipine, nimodipine, nisoldipine, nitrendipine, perhexiline, tipamil, verapamil, pranidipine, their salts, solvates, hydrates, and any mixture thereof.

The following substances can be used as a local anesthetic: proxymetacaine, bupivacaine, benzocaine, bromoaniline dimethylamino propane acid, articaine, oxybuprocaine, lidocaine, tetracaine, dyclonine, dimethylaminoethyl ester of n-butylamino benzoic acid, mepivacaine, ropivacaine, procaine, trimecaine or a mixture thereof.

The pharmacological composition may additionally comprise from 0 to 5 wt % of an antibiotic, an imidazole derivative, for example, metronidazole, tinidazole, clotrimazole, secnidazole, ornidazole and others.

One of the advantages of this pharmaceutical composition for treating hemorrhoids and anal fissure is a synergistic effect between its active components and achievement of a rapid therapeutic effect as compared to known analogues, since action of a calcium channel blocker ensures local vasodilator effect and, as a consequence, a faster penetration of anesthetic and wound healing agent to the affected areas.

Any fillers can be used as excipients in the pharmaceutical composition to create a pharmaceutical composition in the form of a gel, ointment, suppositories, cream, suspended gel, lotion, for example water, polyethylene 400, propylene glycol, dimethylsulfoxide, xanthanes, cellulose and its derivatives, vegetable oils, petrolatum, wax, polyvinylpyrrolidone, carbomers and their salts, thiethanolamine, benzalkonium chloride and others.

The pharmacological composition may contain gelling agents, stabilizers and preservatives.

Hydroxyethyl cellulose or sodium carboxymethyl cellulose or hydroxypropyl cellulose are used as a gelling base (gelling agent).

Paraben sodium salts compatible with active substances and well-proven, which meet the requirements of pharmacopoeia (methylparaben, propylparaben, etc.) are selected as preservatives. Other preservatives, such as propylene glycol and the like can also be used.

Sodium salt of ethylenediaminetetraacetic acid used in the pharmaceutical industry can be used as a stabilizer, an analogue is TRILON-B or the other, similar in properties and effect.

### Embodiment of the Invention

Embodiments of the invention are illustrated by the following examples:

### Example 1.

Preparation of gel comprising 3.3 wt % methyluracil, 0.13 wt % nifedipine, 1.3 wt % lidocaine.

Step-by-step procedure for preparing 1000 g of gel is given below.
1. The gel is prepared by mixing 33 grams of methyluracil, 13 grams of lidocaine, two grams of Carbopol (a mixture of carbomers), one gram of ethylenediaminetetraacetic acid (EDTA), two grams of Nipagin (propylparaben, antimicrobial additive, preservative) and 18.5 grams of triethanolamine with addition of water to total weight of 850 grams.
2. 1.3 grams of nifedipine were mixed with 10 ml of 96% ethanol. 40 grams of polyethylene glycol-400 were added to the obtained solution. After thorough mixing, propylene glycol was added to the obtained solution to a total weight of 150 grams.
3. The nifedipine solution was added with thorough mixing to the suspended gel comprising methyluracil and lidocaine to form a pharmaceutical gel composition.

Test results of using a gel comprising 3.3 wt % methyluracil, 0.13 wt % nifedipine, 1.3 wt % lidocaine in patients suffering from hemorrhoids and anal fissures.

100 tubes containing 50 grams of the gel prepared according to Example 1 were distributed to patients suffering from hemorrhoids and anal fissures. 100 patients applied the gel 2-3 times daily for 21 to 45 days. As a result of the gel application, recovery was observed in all cases (ease from pain, cessation of bleeding, elimination or significant reduction of hemorrhoids edema, healing of anal fissures). No side effects were observed at the gel application. The majority of patients (approximately 58%) recover within 21 to 28 days, while the remaining 42% of patients recover within 29 to 45 days. At the same time, the majority of patients (about 60%) noted attenuation of pain sensations already within the first three days of the gel application.

### Example 2.

Preparation of a gel comprising 5 wt% methyluracil, 0.2 wt % nifedipine, 2 wt% lidocaine.

Step-by-step procedure for preparing 1000 g of gel is given below.
1. The gel is prepared by mixing 50 grams of methyluracil, 20 grams of lidocaine, two grams of Carbopol (a mixture of carbomers), one gram of ethylenediaminetetraacetic acid (EDTA), two grams of Nipagin (propylparaben, antimicrobial additive, preservative) and 18.5 grams of triethanolamine with addition of water to total weight of 850 grams.
2. 2 grams of nifedipine were mixed with 10 ml of 96 % ethanol. 40 grams of polyethylene glycol-400 were added to the obtained solution. Thorough mixing the solution. Propylene glycol was added to the obtained solution to a total weight of 150 grams.
3. The nifedipine solution was added with thorough mixing to the suspended gel comprising methyluracil and lidocaine to form a pharmaceutical gel composition.

Test results of using a gel comprising 5 wt % methyluracil, 0.2 wt % nifedipine and 2 wt % lidocaine in patients suffering from hemorrhoids and anal fissures.

100 tubes containing 50 grams of the gel prepared according to Example 2 were distributed to patients suffering from hemorrhoids and anal fissures. 100 patients applied the gel 2-3 times daily for 21 to 45 days. As a result of the gel application, recovery was observed in all cases (ease from pain, cessation of bleeding, elimination or significant reduction of hemorrhoids edema, healing of anal fissures). No side effects were observed at the gel application. When applying the gel prepared in accordance with example 2, the majority of patients (approximately 66 %) recover within 21 to 28 days, while the remaining 34% of patients recover within 29 to 45 days. At the same time, the majority of patients (about 76 %) noted attenuation of pain sensations within the first three days of the gel application.

### Example 3.

Preparation of gel comprising 7.5 wt % methyluracil, 0.3 wt % nifedipine, 5 wt % lidocaine.

Step-by-step procedure for preparing 1000 g of gel is given below.
1. the gel is prepared by mixing 75 grams of methyluracil, 30 grams of lidocaine, two grams of Carbopol (a mixture of carbomers), one gram of ethylenediaminetetraacetic acid (EDTA), two grams of Nipagin (propylparaben, antimicrobial additive, preservative) and 18.5 grams of triethanolamine with addition of water to total weight of 850 grams.
2. 3 grams of nifedipine were mixed with 10 ml of 96% ethanol. 40 grams of polyethylene glycol-400 were added to the obtained solution. After thorough mixing, propylene glycol was added to the obtained solution to a total weight of 150 grams.
3. The nifedipine solution was added with thorough mixing to the suspended gel comprising methyluracil and lidocaine to form a pharmaceutical gel composition.

Test results of using a gel comprising 7.5 wt % methyluracil, 0.3 wt % nifedipine and 5 wt % lidocaine in patients suffering from hemorrhoids and anal fissures.

100 tubes containing 50 grams of the gel prepared according to Example 3 were distributed to patients suffering from hemorrhoids and anal fissures. 100 patients applied the gel 2-3 times daily for 21 to 45 days. As a result of the gel application, recovery was observed in all cases (ease from pain, cessation of bleeding, elimination or significant reduction of hemorrhoids edema, healing of anal fissures). No side effects were observed at the gel application. When applying the gel prepared in accordance with example 3, the majority of patients (approximately 64 %) recover within 21 to 28 days, while the remaining 36 % of patients recover within 29 to 45 days. Moreover, approximately 81% of patients noted attenuation of pain sensations within the first three days of the gel application.

### Comparative example 4.

A gel was prepared according to the closest analogue (RU 2592366) comprising 3.3 wt % methyluracil, 0.13 wt % isosorbide dinitrate, 0.13 wt % nifedipine, 1.3 wt % lidocaine.

Step-by-step procedure for preparing 1000 g of gel is given below.
1. The gel is prepared by mixing 33 grams of methyluracil, 13 grams of lidocaine, two grams of Carbopol (a mixture of carbomers), one gram of ethylenediaminetetraacetic acid (EDTA), two grams of Nipagin (propylparaben, antimicrobial additive, preservative) and 18.5 grams of triethanolamine with addition of water to total weight of 850 grams.
2. 1.3 grams of nifedipine were mixed with 10 ml of 96% ethanol. 40 grams of polyethylene glycol-400 were added to the resulting solution. After thorough mixing, propylene glycol was added to the resulting solution to a total weight of 135 grams.
3. 10 grams of dimethisulfoxide (DMSO) were added to five grams of a mixture comprising 26 wt % (1.3 grams) isosorbide dinitrate and 74 wt % (3.7 grams) lactose. The mixture was stirred until a clear solution was obtained.
4. Isosorbide dinitrate solution was added to the nifedipine solution.
5. The combined solution of isosorbide dinitrate and nifedipine was added with thorough mixing to the suspended gel comprising methyluracil and lidocaine to form a pharmaceutical gel composition.

Test results of using a gel comprising 3.3 wt % methyluracil, 0.13 wt % isosorbide dinitrate, 0.13 wt % nifedipine, 1.3 wt % lidocaine in patients suffering from hemorrhoids and anal fissures.

100 tubes containing 50 grams of the gel prepared according to Example 1 were distributed to patients suffering from hemorrhoids and anal fissures. 100 patients applied the gel 2-3 times daily for 21 to 45 days. As a result of the gel application, recovery was observed in all cases (ease from pain, cessation of bleeding, elimination or significant reduction of hemorrhoids edema, healing of anal fissures). No side effects were observed at the gel application. The majority of patients (approximately 61%) recover within 21 to 28 days, while the remaining 39% of patients recover within 29 to 45 days. Moreover, only 59 % of patients noted attenuation of pain sensations within the first three days of the gel application.

Thus, preparation of the gel according to the proposed formulation, as compared to the closest analogue, enables to simplify the medication preparation procedure, reducing it by two operations and, accordingly, to reduce the composition preparation time, and also to reduce its cost.

Moreover, use of the proposed composition, as compared to the closest analogue, has only a slightly reduced objective observed therapeutic effect, which, nevertheless, significantly exceeds the effect of the other known medications.

Besides, when using this medication, patients noted a faster and more significant attenuation of pain sensations, which also has a positive effect on general well-being and contributes to a faster recovery.

When using a comparative composition not comprising methyluracil, distribution of recovered patients by treatment time was of dissimilar nature. Thus, the majority of patients (approximately 59%), among the patients recovered up to 45 days, 60% of patients used the comparative composition not comprising methyluracil, recovered over a longer period of 29 to 45 days. With an increase in duration of treatment with a comparative composition over 45 days, the total percentage of patients with complete recovery increases even more. Moreover, only 47% of patients noted attenuation of pain sensations within the first 3 days of using the medication.

Based on the test results, the optimal ratio of the components forming the composition has been determined. According to the studies, when decreasing or increasing the concentration of active components from the claimed limits, no enhancement of the composition therapeutic properties occurs. That is, any deviations from the optimal ratio of the components do not allow to obtain the desired therapeutic effect. Moreover, the qualitative composition of the claimed composition is selected empirically and absence of any ingredient in its composition does not allow to achieve the result obtained with the use of the claimed composition.

Thus, use of a complex pharmacological composition based on 3.3-7.5 wt % methyluracil, 0.13-0.3 wt % nifedipine and 1.3-5 wt % lidocaine, in patients suffering from hemorrhoids and anal fissures, has enabled to enhance and accelerate achieving the therapeutic effect, as compared to the composition not comprising methyluracil.

Other interpretations and variations of the present invention are possible, which do not contradict the claims in terms of preparing a pharmaceutical composition, type of the pharmaceutical composition, method and scope of its application. In particular, based on the proposed composition, including 3.3-7.5 wt % methyluracil, 0.13-0.3 wt % nifedipine and 1.3-5 wt % local anesthetic there could be prepared ointments, suppositories, creams, lotions, suspensions that can be applied locally for treating chronic hemorrhoids, acute hemorrhoidal manifestations (all stages), anal fissures, traumatic injuries of anus and rectum, sphincter, post-operative care of persons completed a treatment of hemorrhoids, polyps, papillomas, condylomata by surgical methods, pathological changes of hemorrhoidal boluses, as anesthetic and relaxing agent for anorectal area, anal mucous membrane irritation, sphincter spasmolysis, in mono- and complex therapy of proctologic diseases, such as: proctitis, periproctitis, rectum cancer, anal cancer, proctosigmoiditis, anal fistulas, anal inflammatory processes, cryptitis, megacolon, rectal fibroma.

## Claims

1. A pharmacological composition for treating proctologic diseases, **characterized in that** it comprises 3.3-7.5 wt% methyluracil, 0.13-0.3 wt% nifedipine, 1.3-5 wt% lidocaine, a pharmacologically acceptable carrier - rest.

2. The pharmacological composition according to claim 1, **characterized in that** it could be in the form of gel, or ointment, or cream, or suppositories, or suspended gel, or lotion, or emulsion.

3. A pharmacological composition for treating proctologic diseases, **characterized in that** it consists of 5 wt% methyluracil, 0.2 wt% nifedipine, 2-5 wt% lidocaine, a pharmacologically acceptable carrier - rest.

4. The pharmacological composition according to claim 3, **characterized in that** it could be in the form of gel, or ointment, or cream, or suppositories, or suspended gel, or lotion, or emulsion.
